# EUROPEAN PATENT APPLICATION

(11) **EP 3 287 439 A1**
(43) Date of publication of application: **28.02.2018**
(21) Application number: 16820142.4
(22) Date of filing: 01.07.2016
(51) Int. Cl.: C07C 303/02, C07C 309/14

(54) **METHOD FOR PREPARING TAURINE**

(30) Priority: 28.06.2016 CN 201610489140
(71) Applicant: QIANJIANG YONGAN PHARMACEUTICAL CO., LTD, Economy Development Zone Qianjiang, Hubei 433132 (CN)
(72) Inventor: CHEN, Yong, Qianjiang Hubei 433132 (CN); FANG, Xiquan, Qianjiang Hubei 433132 (CN); LI, Shaobo, Qianjiang Hubei 433132 (CN); JIANG, Xiaojun, Qianjiang Hubei 433132 (CN)
(74) Representative: Ter Meer Steinmeister & Partner
(86) International application number: PCT/CN2016/088077
(87) International publication number: WO 2018/000404

(57) **Abstract**

The present application provides a process for producing taurine, comprising the steps as follows: (a) mixing isethionic acid with taurine salt solution until the system pH reaches a certain value in a range from 5.0 to 9.5; (b) separating liquid phase and solid phase of the system; wherein said solid phase is the crude product of taurine, and said liquid phase is isethionate solution; (c) reacting ammonia solution with said liquid phase obtained from step (b) to obtain taurine salt solution. It uses isethionic acid to adjust the pH of the taurine salt solution, avoiding the problem causing by using sulphate acid to adjust the pH in the traditional process. By the recycling use of the cations in taurine salts, a new raw material or reagent does not need to be added which is benefit to reducing the use of dangerous chemical materials, simplifying the production process greatly, improving the utilization rate of raw materials, increasing the yield of the product and decreasing production cost significantly.

## Description

### Technical Field

The present application relates to a process for the preparation of taurine, which belongs to the field of drug synthesis.

### Technical Background

Taurine is a non-protein amino acid and a drug with functions of anti-inflammatory, febrifuge, analgesic, anticonvulsant, lowering blood pressure and as the like. Taurine has beneficial effects on brain growth, nerve conduction, improvement of visual function, and calcium absorption of infants. Taurine also has a series of distinctive functions on cardiovascular system. Moreover, it can help one build up health and relieve fatigue. Thus, taurine is widely used in the fields of health care, food health and as the like.

Taurine can be prepared by extraction from biology and chemical synthesis. The former is affected by raw materials and cost, having been rarely used. In the current industrial production, taurine is produced either through the esterification of ethanolamine or by ethylene oxide. Compared with the esterification of ethanolamine, the ethylene oxide process is preferred due to its low cost, high quality product, low environmental pollution, being synthesized continuously and as the like.

The ethylene oxide process is developed as a new process with the reaction mechanism as follows:

NaOH + SO₂ → NaHSO₃

NaHSO₃ + CH₂CH₂O → HOCH₂CH₂SO₃Na

HOCH₂CH₂SO₃Na + NH₃ → H₂NCH₂CH₂SO₃Na + H₂O

2H₂NCH₂CH₂SO₃Na + H₂SO₄ → 2H₂NCH₂CH₂SO₃H + Na₂SO₄

However, there still are some drawbacks in the ethylene oxide process which are listed as follows: ① A large amount of sulfuric acid and liquid solutions of bases are used, which will finally convert into sodium sulphate. With accumulation of time, part of taurine will be taken away by sodium sulphate, causing a loss of material. In the meanwhile, because sodium sulphate always exists in the mother liquor, it is inevitable that there is a residue of sulphates in the crude product which is obtained by being separated centrifugally, finally leading to an overproof phenomenon of sulphates in product. And due to the existence of sodium sulphates in the mother liquor, the cooler, the heater, the synthesis reactor and the high-pressure pipeline are easily blocked under the synthesis conditions of high temperature and high pressure, effecting the normal production. Moreover, it is difficult to treat the massive solid waste of sulphates with the increase in labor intensity. ② The existence of sodium sulphate produced by neutralization reaction effects the first extraction rate, making the multiple extraction necessary to obtain taurine (H₂NCH₂CH₂SO₃H) in the following extraction process, bring a great deal of waste mother liquor and causing material waste low yield and pollution to the environment. And during the concentration of the mother liquid, it need consume plenty of steam resulting in high energy consumption. ③ The production of massive solid waste of sulphates brings a great pressure to environmental protection.

Thus, it is imperative to seek an effective method to solve the problems presented in the current process for producing taurine.

### Summary of the Invention

The present application provides a process for producing taurine. Isethionic acid is used to adjust the pH of taurine salt solution, to avoid the problems caused by adjusting the pH using sulfuric acid in a conventional process. By the recycling use of the cations in taurine salts, a new raw material or reagent does not need to be added which is benefit to reducing the use of dangerous chemical materials, simplifying the production process greatly, improving the utilization rate of raw materials, increasing the yield of the product and decreasing production cost significantly.

The process for producing taurine, comprising the steps as follows:
(a) mixing isethionic acid with taurine salt solution until the system pH reaches a certain value in a range from 5.0 to 9.5;
(b) separating liquid phase and solid phase of the system; wherein said solid phase is the crude product of taurine, and said liquid phase is isethionate solution;
(c) reacting ammonia solution with said liquid phase obtained from step (b) to obtain taurine salt solution.

Preferably, said taurine salt is at least one selected from ammonium salts of taurine, alkali metal salts of taurine, alkaline earth salts of taurine. And the type of isethionate in step (b) is corresponding to the type of taurine salt, which is at least one selected from ammonium salts of hydroxyethyl sulfonate, alkali metal salts of hydroxyethyl sulfonate, alkaline earth salts of hydroxyethyl sulfonate. More preferably, said taurine salt is sodium taurine and said isethionate is sodium isethionate.

Preferably, in step (a), said taurine salt solution is obtained by dissolving taurine salt in water and/or recycling of said taurine salt solution obtained in step (b).

Preferably, in step (a), the mass percent concentration of taurine salt in said taurine salt solution is in a range from 35wt% to 60wt%. More preferably, in step (a), the mass percent concentration of taurine salt in said taurine salt solution is in a range from 40wt% to 50wt%.

Preferably, step (a) is that mixing isethionic acid with taurine salt solution until the system pH reaches a certain value in a range from 5.5 to 9.0. More preferably, the low limit of the pH range is selected from 5.5, 6.0, 6.5, 7.0, 7.5 or 7.8; and the high limit of the pH range is selected from 9.0, 8.5 or 8.0.

With respect to the temperature of mixing isethionic acid with the taurine salt solution in step (a), one skilled in the art can select the temperature at a range from 20°C to 80°C according to the actual demand. In general, mixing isethionic acid with the taurine salt solution in step (a) is carried out at room temperature.

Preferably, in step (b), separation of liquid phase and solid phase of the system is carried out at a temperature range from 0°C to 50°C. More preferably, in step (b), separation of liquid phase and solid phase of the system is carried out at a temperature range from 10°C to 20°C.

In step (b), the mass percent concentration of taurine in said crude product of taurine is ≥80%.

According to the actual demand, one skilled in the art can select the separation method in step (b).

Preferably, step (c) is that adding ammonia solution into said liquid phase obtained from step (b) and reacting for 0.5-2h at a reaction temperature range from 200°C to 280°C and a reaction pressure range from 10 MPa to 21 MPa to obtain taurine salt solution. More preferably, said reaction temperature range in step (c) is from 250°C to 260°C.

As an embodiment, said reaction pressure range in step (c) is from 10MPa to 15MPa.

As an embodiment, said reaction pressure range in step (c) is from 15MPa to 21MPa.

Preferably, in step (c), the volume percentage of ammonia solution in the mixture which is obtained by adding ammonia solution into said liquid phase obtained from step (b) is from 18% to 30%.

As an embodiment of the present application, said process for producing taurine is a batch process. The separation method in step (b) is selected from suction filtration, filtration or intermittent centrifugation

As an embodiment of the present application, said process for producing taurine is a continuous process. The separation method in step (b) is continuous separation of the liquid-solid phase, which includes, but is not being limited to, continuous sedimentation separation, hydrocyclone separation, continuous filtration separation and continuous centrifugal separation.

The beneficial effects of the present applicant include, but are not limited to:
(1) The process for producing taurine provided in the present application uses isethionic acid to adjust the pH of the taurine salt solution, avoiding the problem causing by using sulphate acid to adjust the pH in the traditional process, reducing the use of hazardous chemicals raw materials. In the meanwhile, it avoids generating a large number of waste solid salts such as sodium sulfate which completely solves overproof probelom of sulphates in taurine product produced by the traditional process.
(2) The process for producing taurine provided in the present application does not introduce new raw materials or agents, complying with chemical atomic economy. In the meanwhile, it solves some problems of process, quality and environmental protection in the conventional process and it significantly reduces production costs which can provide a strong support for the further development of the enterprise.
(3) The process for producing taurine provided in the present application greatly simplifies the production process, avoiding the problem of environmental pollution caused by discharging the part of mother taurine solution which is not used sufficiently in the multiple concentration process of mother taurine solution. It reduces the usage amount of steam in the multiple concentration process of mother taurine solution, improving the utilization ratio of raw materials and the yield of product.
(4) The process for producing taurine provided in the present application can be carried out in a continuous manner which is benefit to the large-scale industrial production.

### Description of the Figures

Figure 1 shows a schematic flowchart of an embodiment provided by the present application for the production of taurine.
Figure 2 shows a schematic flowchart of another embodiment provided by the present application for the production of taurine.

### Detailed Description of the Embodiment

The following examples will illustrate the practice of the present invention by combining with the Figures but are not intended to limit its scope.
In the examples, all reagents are purchased commercially if there is no special illustration.

In the examples, taurine was determined with LC10AT High Performance Liquid Chromatography (HPLC) produced by Shimadzu Company; sodium hydroxyethyl sulfonate was determined with ICS900 Ion Chromatography produced by Dionex Corporation.

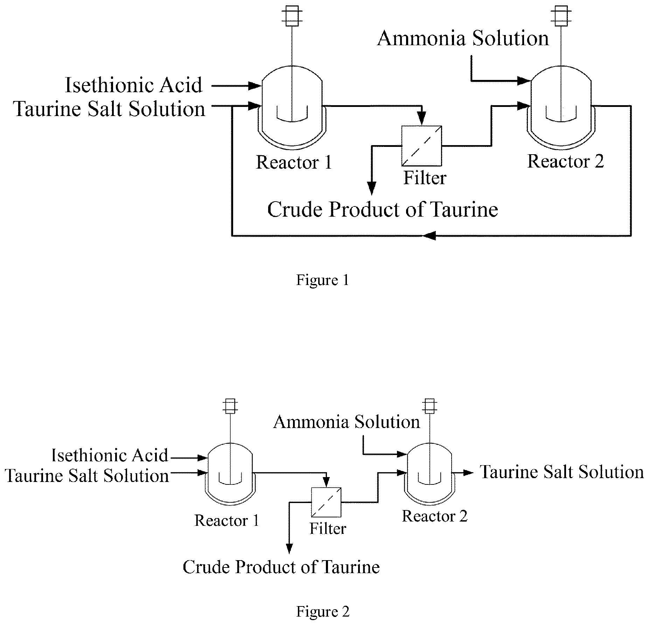

Figure 1 shows a schematic flowchart of an embodiment provided by the present application for the production of taurine which is a continuous process for producing taurine. Reactor 1 and Reactor 2 both are Continuous Stirred Tank Reactor (abbreviated as CSTR). Isethionic acid and taurine salt solution as the materials were added into Reactor 1; and the pH was adjusted to a certain value in a range from 5.0 to 9.5 by adjusting each amount of isethionic acid and taurine salt solution. The solid-liquid phase materials which were exported from Reactor 1 entered into a filter; after being filtered, the solid phase was obtained as the crude product of taurine and the liquid phase passing through the filter (isethionate solution) entered into Reactor 2. Reactor 2 was a Pressurized Reactor. The isethionate solution passing through the filter reacted with ammonia solution in Reactor 2 to obtain taurine salt solution which was recycled back to Reactor 1.

Figure 2 shows a schematic flowchart of another embodiment provided by the present application for the production of taurine which is a continuous process or a batch process for producing taurine. Reactor 1 and Reactor 2 are Continuous Stirred tank Reactor or Batch Tank Reactor. Isethionic acid and taurine salt solution as the materials were added into Reactor 1; and the pH was adjusted to a certain value in a range from 5.0 to 9.5 by adjusting each amount of isethionic acid and taurine salt solution. When Reactor 1 was a Continuous Stirred Tank Reactor, the materials were continuously fed into Reactor 1 and the solid-liquid phase materials were exported continuously, entering into a filter continuously and being filtered continuously. When Reactor 1 is a Batch Tank Reactor, all of the solid-liquid phase materials were taken out from Reactor 1 after the reaction was completed, entering into a filter to be intermittently filtered; after being intermittently filtered, the solid phase was obtained as the crude product of taurine and the liquid phase passing through the filter (isethionate solution) entered into Reactor 2. Reactor 2 was a Pressurized Reactor. The isethionate solution passing through the filter reacted with ammonia solution in Reactor 2 to obtain taurine salt solution which could be kept as the product or also could be recycled back to Reactor 1 as a material for reacting again.

### Example 1

500 mL of aqueous sodium taurine solution with mass percent concentration of 46wt% was added into a reaction bottle, and then isethionic acid was added slowly under stirring at room temperature until the pH reached to 5.5. The system was filtered to obtain 216g of the crude product of taurine after the system temperature was dropped to 15°C. The filtrate was transferred into a reaction bottle, and then 1710 g of ammonia solution with mass percent concentration of 26.5% was mixed with the above filtrate. In autoclave, the reaction was carried out at 250°C and 10 MPa pressure for 1 h. 697g of sodium taurine solution was obtained after cooling, discharging and subsequently removing ammonia by evaporation. The content of taurine was determined by HPLC. The content of sodium isethionate was determined by Ion Chromatography. The results were shown in Table 1; wherein the percentages in the table all were mass percentage.

**Table 1**

| Each component content in the crude product of taurine | Content of sodium taurine in the sodium taurine solution |
|---|---|
| Content of taurine: 87.88% | Content of sodium taurine: 46.15% |
| Content of water: 6.92% | |
| Content of sodium isethionate: 4.40% | |

### Example 2

500 mL of sodium taurine solution obtained in Example 1 was added into a reaction bottle, and then isethionic acid was added slowly under stirring at room temperature until the pH reached to 7.5. The system was filtered to obtain 229g of the crude product of taurine after the system temperature was dropped to 15°C. The filtrate was transferred into a reaction bottle, and then 1700 g of ammonia solution with mass percent concentration of 25.8% was mixed with the above filtrate. In autoclave, the reaction was carried out at 253°C and 10.5 MPa pressure for 1 h. 615g of sodium taurine solution was obtained after cooling, discharging and subsequently removing ammonia by evaporation. The content of taurine was determined by HPLC. The content of sodium isethionate was determined by Ion Chromatography. The results were shown in Table 2; wherein the percentages in the table all were mass percentage.

**Table 2**

| Each component content in the crude product of taurine | Content of sodium taurine in the sodium taurine solution |
|---|---|
| Content of taurine: 88.64% | Content of sodium taurine: 46.35% |
| Content of water: 6.34% | |
| Content of sodium isethionate: 4.42% | |

### Example 3

500 mL of sodium taurine solution with mass percent concentration of 46wt% was added into a reaction bottle, and then isethionic acid was added slowly under stirring at room temperature until the pH reached to 8.5. The system was filtered to obtain 223g of the crude product of taurine after the system temperature was dropped to 15°C. The filtrate was transferred into a reaction bottle, and then 1690 g of ammonia solution with mass percent concentration of 26.0% was mixed with the above filtrate. In autoclave, the reaction was carried out at 256°C and 10 MPa pressure for 1 h. 580g of sodium taurine solution was obtained after cooling, discharging and subsequently removing ammonia by evaporation. The content of taurine was determined by HPLC. The content of sodium isethionate was determined by Ion Chromatography. The results were shown in Table 3; wherein the percentages in the table all were mass percentage.

**Table 3**

| Each component content in the crude product of taurine | Content of sodium taurine in the sodium taurine solution |
|---|---|
| Content of taurine: 90.82% | Content of sodium taurine: 46.10% |
| Content of water: 5.68% | |
| Content of sodium isethionate: 3.0% | |

### Example 4

500 mL of aqueous sodium taurine solution with mass percent concentration of 46wt% was added into a reaction bottle, and then isethionic acid was added slowly under stirring at room temperature until the pH reached to 9.0. The system was filtered to obtain 201g of the crude product of taurine after the system temperature was dropped to 15°C. The filtrate was transferred into a reaction bottle, and then 1680 g of ammonia solution with mass percent concentration of 25.9% was mixed with the above filtrate. In autoclave, the reaction was carried out at 258°C and 11 MPa pressure for 1 h. 585g of sodium taurine solution was obtained after cooling, discharging and subsequently removing ammonia by evaporation. The content of taurine was determined by HPLC. The content of sodium isethionate was determined by Ion Chromatography. The results were shown in Table 4; wherein the percentages in the table all were mass percentage.

**Table 4**

| Each component content in the crude product of taurine | Content of sodium taurine in the sodium taurine solution |
|---|---|
| Content of taurine: 91.5% | Content of sodium taurine: 45.80% |
| Content of water: 4.93% | |
| Content of sodium isethionate: 3.07% | |

### Example 5

600 mL of sodium taurine solution obtained in Example 2 with mass percent concentration of 46.35% was added into a reaction bottle, and then isethionic acid was added slowly under stirring at room temperature until the pH reached to 7.8. The system was filtered to obtain 221g of the crude product of taurine after the system temperature was dropped to 15°C. The filtrate was transferred into a reaction bottle, and then 1700 g of ammonia solution with mass percent concentration of 25.9% was mixed with the above filtrate. In autoclave, the reaction was carried out at 258°C and 11 MPa pressure for 1.5 h. 605g of sodium taurine solution was obtained after cooling, discharging and subsequently removing ammonia by evaporation. The content of taurine was determined by HPLC. The content of sodium isethionate was determined by Ion Chromatography. The results were shown in Table 5; wherein the percentages in the table all were mass percentage.

**Table 5**

| Each component content in the crude product of taurine | Content of sodium taurine in the sodium taurine solution |
|---|---|
| Content of taurine: 89.5% | Content of sodium taurine: 46.25% |
| Content of water: 6.03% | |
| Content of sodium isethionate: 3.42% | |

It will be understood that the foregoing Examples are only some examples of the present application, but not limited to the application in any form. Although the optimized examples of the present application are illustrated as above, they do not limit the application. In view of the instant disclose various modifications of the present application will be self-evident to those skilled in the art and are to be included within the spirit and purview of the present application and the scope of the appended claims.

## Claims

1. A process for producing taurine, comprising the steps as follows:
(a) mixing isethionic acid with taurine salt solution until the system pH reaches a certain value in a range from 5.0 to 9.5;
(b) separating liquid phase and solid phase of the system; wherein said solid phase is the crude product of taurine, and said liquid phase is isethionate solution;
(c) reacting ammonia solution with said liquid phase obtained from step (b) to obtain taurine salt solution.

2. The process according to claim 1, wherein said taurine salt is at least one selected from ammonium salts of taurine, alkali metal salts of taurine, alkaline earth salts of taurine.

3. The process according to claim 1, wherein in step (a), said taurine salt solution is obtained by dissolving taurine salt in water and/or recycling of said taurine salt solution obtained in step (b).

4. The process according to claim 1, wherein in step (a), the mass percent concentration of taurine salt in said taurine salt solution is in a range from 35wt% to 60wt%.

5. The process according to claim 1, wherein in step (a), the mass percent concentration of taurine salt in said taurine salt solution is in a range from 40wt% to 50wt%.

6. The process according to claim 1, wherein step (a) is that mixing isethionic acid with taurine salt solution until the system pH reaches a certain value in a range from 5.5 to 9.0.

7. The process according to claim 1, wherein in step (b), separation of liquid phase and solid phase of the system is carried out at a temperature range from 0°C to 50°C.

8. The process according to claim 1, wherein in step (b), separation of liquid phase and solid phase of the system is carried out at a temperature range from 10°C to 20°C.

9. The process according to claim 1, wherein said process for producing taurine is a batch process.

10. The process according to claim 1, wherein said process for producing taurine is a continuous process.
